Europäisches Patentamt

European Patent Office

Office européen des brevets

(19)

(11) Veröffentlichungsnummer: **0 139 608**
**B1**

(12) **EUROPÄISCHE PATENTSCHRIFT**

(45) Veröffentlichungstag der Patentschrift:
**01.06.88**

(21) Anmeldenummer: **84730108.2**

(22) Anmeldetag: **10.10.84**

(51) Int. Cl.⁴: **A 61 K 31/565** // (A61K31/565, 31:557)

(54) Prostaglandine und Antigestagene zur Geburtseinleitung oder für den Schwangerschaftsabbruch.

(30) Priorität: **12.10.83 DE 3337450**

(43) Veröffentlichungstag der Anmeldung:
**02.05.85 Patentblatt 85/18**

(45) Bekanntmachung des Hinweises auf die Patenterteilung:
**01.06.88 Patentblatt 88/22**

(84) Benannte Vertragsstaaten:
**AT BE CH DE FR GB IT LI LU NL SE**

(56) Entgegenhaltungen:
**EP - A - 0 057 115**
**DE - A - 2 528 419**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Elger, Walter, Dr., Schorlemerallee 12B,**
**D-1000 Berlin 33 (DE)**
Erfinder: **Beier, Sybille, Dr., Uhlandstrasse 121,**
**D-1000 Berlin 31 (DE)**

Beschreibung

Die Erfindung betrifft ein Kombinationserzeugnis zur gemeinsamen Verwendung zur Geburtseinleitung oder für den Schwangerschaftsabbruch, enthaltend ein Prostaglandin (PG) und ein Antigestagen (AG) sowie die Verwendung von Prostaglandinen zur Geburtseinleitung oder für den Schwangerschaftsabbruch in Kombination mit Antigestagenen.

Um Gefahren für Mutter und/oder Kind abzuwenden, ist es manchmal erforderlich, eine Geburt künstlich einzuleiten oder eine Schwangerschaft vorzeitig zu beenden.

Dafür stehen chirurgische Techniken und pharmakologische Methoden zur Verfügung.

Eine günstige pharmakologische Methode ist die vaginale oder intramuskuläre Applikation von Prostaglandinen, die im Falle des Schwangerschaffsabbruchs im 1. oder 2. Schwangerschaftstrimesters (Contraception 1983, Vol. 27, 51–60 und Int. J. Gynäcol. Obstet. 1982, Vol. 20, 383–386) vorgenommen wird.

Vorteile der Prostaglandine sind ihre einfache Anwendbarkeit und ihre Einsatzmöglichkeit über einen langen Zeitraum der Schwangerschaft. Als Nachteile der Prostaglandine sind akute Nebenwirkungen wie Schmerzen und Übelkeit zu nennen, ausserdem liegt die Erfolgsrate im Falle des Schwangerschaftsabbruches in fortgeschrittenen Phasen der Schwangerschaft auch bei einer längeren Dauer der Prostaglandinbehandlung nicht über 90%.

Zur Geburtseinleitung sind auch Kombinationen von Prostaglandinen mit Östrogenen vorgeschlagen worden (DE-A-2 528 419); sie haben sich jedoch klinisch nicht durchsetzen können.

Eine andere Möglichkeit der Beendigung einer Schwangerschaft besteht in der Applikation eines Antigestagens (Med. et Hyg. 1982, Vol. 40, 2087–2093). Antigestagene sind besser verträglich als Prostaglandine, haben aber eine grössere Latenz und individuelle Variabilität des Wirkungseintritts im Vergleich mit den Prostaglandinen.

Es wurde nun gefunden, dass PG-typische und AG-typische Nachteile vermieden werden, wenn man Prostaglandine (PG) und Antigestagene (AG) gemeinsam für den Schwangerschaftsabbruch verwendet.

Die Gewichtsmengen an Prostaglandin und Antigestagen können bei gemeinsamer Anwendung im Vergleich zu den üblichen Mengen stark herabgesetzt werden, wobei die Erfolgsrate der Schwangerschaftsabbrüche sogar noch gesteigert werden kann. Prostaglandin und Antigestagen werden vorzugsweise getrennt, gleichzeitig und/oder zeitlich abgestuft (sequential), in einem Gewichtsverhältnis von Prostaglandin zu Antigestagen von im wesentlichen 1:20 bis 1:6000 verwendet.

Als für die erfindungsgemässe Verwendung geeignete Prostaglandine kommen alle für den Schwangerschaftsabbruch geeignete Prostaglandine infrage; das sind insbesondere Prostaglandine der E- und F-Reihe. Beispielsweise genannt seien:
Prostaglandine $E_2$,
Prostaglandin $F_{2\alpha}$,
Prostaglandin E-Derivate, wie
16-Phenoxy-ω-17,18,19,20-tetranor-$PGE_2$-methylsulfonylamid (Sulproston),
16,16-Dimethyl-trans-$\Delta^2$-$PGE_1$-methylester (Gemeprost),
9-Deoxo-16,16-dimethyl-9-methylen-$PGE_2$ (Metenenprost),
Prostaglandin F-Derivate, wie
15-Methyl-PG $F_{2\alpha}$-methylester
(5Z,13E)-(9R,11R,15R)-9-Chlor-11,15-dihydroxy-16,16-dimethyl-5,13-prostadiensäure (DE-OS 29 50 027),
(5Z,13E)-(9R,11R,15R)-11,15-Dihydroxy-9-fluor-16-phenoxy-17,18,19,20-tetranor-5,13-prostadiensäure (DE-OS 31 26 924),
(5Z,13E)-(9R,11R,15R)-11,15-Dihydroxy-16,16-dimethyl-9-fluor-5,13-prostadiensäure (DE-OS 31 26 924),
(5Z,13E)-(9R,11R,15R)-9-Brom-11,15-dihydroxy-16-phenoxy-17,18,19,20-tetranor-5,13-prostadiensäure (DE-OS 31 48 743),
(5Z,13E)-(9R,11R,15R)-9-Brom-11,15-dihydroxy-16,16-dimethyl-5,13-prostadiensäure (DE-OS 31 48 743).

Die Prostaglandine werden in Mengen eingesetzt, die deutlich unterhalb der sonst für den Schwangerschaftsabbruch üblichen Mengen liegen. Die gemäss vorliegender Erfindung anzuwendende Menge hängt u.a. vom Hormonspiegel, vom Alter der Gravidität und der Art der Applikation ab. Bei der Verwendung von Sulproston als Prostaglandin wird man in der Regel mit 0,03 bis 0,5 mg pro Tag auskommen. Die Applikation kann zum Beispiel lokal, topisch, enteral oder parenteral erfolgen. Bei der intramuskulären Injektion bzw. intravenösen Infusion werden beispielsweise Mengen von etwa 0,1 bis 0,3 mg Sulproston pro Tag benötigt. Bei der lokalen Applikation, beispielsweise extraamnial oder intravaginal, werden etwa 0,03 bis 0,5 mg Sulproston pro Tag eingesetzt. Für die topische Anwendung können auch transdermale Systeme wie Hautpflaster eingesetzt werden. Anstelle von Sulproston können erfindungsgemäss biologisch äquivalente Mengen von anderen Prostaglandinen eingesetzt werden.

Die gemeinsame Behandlung mit Prostaglandin und Antigenstagen erfolgt in der Regel über 1 bis 4, vorzugsweise 1 bis 2 Tage, wobei Prostaglandin und Antigestagen vorzugsweise getrennt und gleichzeitig oder getrennt und zeitlich abgestuft (sequential) appliziert werden können.

Bei der Sequenztherapie wird zunächst das Antigestagen über 1 bis 4 Tage und anschliessend das Prostaglandin allein oder Prostaglandin und Antigestagen gemeinsam über 24 Stunden appliziert.

Grundsätzlich können Prostaglandin und Antigestagen auch kombiniert in einer Dosiseinheit appliziert werden.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

11β-[(4-N,N-Dimethylamino)-phenyl]-17β-hydroxy-17α-propinyl-4,9(10)-estradien-3-on (RU-38486) und

11β-[(4-N,N-Dimethylamino)-phenyl]-17β-hydroxy-18-methyl-17α-propinyl-4,9(10)-estradien-3-on und

11β-[(4-N,N-Dimethylamino(-phenyl]-17aβ-hydroxy-17aα-propinyl-D-homo-4,9(10)-16-estratrien-3-on (EP-A-0057115);
ferner

11β-p-Methoxyphenyl-17β-hydroxy-17α-ethinyl-4,9(10)-estradien-3-on (Steroids 37 (1981) 361–382) und

11β-(4-Dimethylaminophenyl)-17α-hydroxy-17β-(3-hydroxypropyl)-13α-methyl-4,9-gonadien-3-on.

Die Antigestagene werden gemäss vorliegender Erfindung in Mengen eingesetzt, die in der Regel unterhalb der sonst für den Schwangerschaftsabbruch üblichen Mengen liegen. Im allgemeinen wird man mit 10–200 mg 11β[(4-N,N-Dimethylamino)-phenyl]-17β-hydroxy-17α-propinyl-4,9(10)-estradien-3-on pro Tag oder einer biologisch äquivalenten Menge eines anderen Antigestagens auskommen.

Die Antigestagene können zum Beispiel lokal, topisch, enteral oder parenteral appliziert werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfchen oder transdermale Systeme wie Hautpflaster infrage.

Eine Dosiseinheit enthält etwa 10 bis 200 mg

11β-[(4-N,N-Dimethylamino)-phenyl]-17β-hydroxy-17α-propinyl-4,9(10)-estradien-3-on

oder eine biologisch äquivalente Menge eines anderen Antigestagens. Die nachstehenden Beispiele sollen die galenische Formulierung erläutern.

Beispiel 1

Zusammensetzung einer gefriergetrockneten Sulproston-Formulierung pro Ampulle

| | | |
|---|---|---|
| 0,1 | mg | Sulproston |
| 5,0 | mg | Polyvinylpyrrolidon (K-Wert = 15–18) |
| 1,95 mg | | Tris(hydroxymethyl)aminomethan-hydrochlorid (Tremetamol · HCl) (aus 1,5 mg Tremetamol und 1 N Salzsäure) |
| 7,05 mg | | |

Zur Dosierung und Anwendung wird der Inhalt der Ampulle mit isotonischer Kochsalzlösung aufgelöst für die intramuskuläre Injektion bzw. intravenöse Infusion oder extraamniale Applikation.

Herstellung der Trockensubstanz

Sulproston wird durch Zugabe einer eisgekühlten Lösung des Polyvinylpyrrolidons und Tremetamols in destilliertem Wasser in Lösung gebracht. Durch Zugabe von 1 N Salzsäure wird unter starker Kühlung der pH-Wert der Lösung auf 5,0 eingestellt. Anschliessend wird die Lösung auf das erforderliche Volumen aufgefüllt. Nach Filtration über ein Membranfilter wird die Lösung in Ampullen dosiert.

Die Lösung wird dann durch Eintauchen der Ampullen in eine Aceton/Trockeneis-Kältemischung eingefroren und sofort in einer vorgekühlten Gefrierstrockenanlage ca. 48 Stunden gefriergetrocknet. Nach Abschluss der Gefriertrocknung werden die Ampullen sofort zugeschmolzen.

Beispiel 2

Zusammensetzung einer Folie mit Sulproston für die vaginale Applikation

| | | |
|---|---|---|
| 0,1 | mg | Sulproston |
| 19,6 | mg | Hydroxypropylcellulose |
| 0,32 mg | | Polyoxyethylenpolyoxypropylenpolymeres (Pluronic F 68[R]) |
| 20,02 mg | | |

Die Folie hat eine Länge von 3 cm.

Beispiel 3

Zusammensetzung einer Folie mit Sulproston für die buccale Applikation

| | | |
|---|---|---|
| 0,3 | mg | Sulproston |
| 9,16 mg | | Hydroxypropylcellulose |
| 9,16 mg | | Cellulosefasern |
| 0,15 mg | | Polyoxyethylenpolyoxypropylenpolymeres (Pluronic F 68[R]) |
| 18,77 mg | | |

Die Fläche der Folie ist 1,2 × 1,2 cm.

Beispiel 4

Zusammensetzung einer Tablette mit Sulproston zur vaginalen Applikation

| | | |
|---|---|---|
| 0,1 mg | | Sulproston |
| 238,9 mg | | Laktose |
| 110,0 mg | | mikrokristalline Cellulose |
| 1,0 mg | | Magnesiumstearat |
| 350,0 mg | | |

Beispiel 5

Zusammensetzung einer weiteren Tablette mit 11β-[(4-N,N-Dimethxylamino)-phenyl]-17β-hydroxy-17α-propinyl-4,9(10)-estradien-3-on zur oralen Applikation

| | |
|---|---|
| 10,0 mg | 11β-[(4-N,N-Dimethylamino)-phenyl]-17β-hydroxy-17α-propinyl-4,9(10)-estradien-3-on |
| 140,5 mg | Laktose |
| 69,5 mg | Maisstärke |

2,5 mg Polyvinylpyrrolidon 25
2,0 mg Aerosil
0,5 mg Magnesiumstearat
225,0 mg Gesamtgewicht

Pharmakologische Beobachtungen

Für die Pilotversuche an graviden Meerschweinchen und Ratten wurden das Prostaglandin Sulproston und die Antigestagene

11β-[(4-N,N-Dimethylamino)-phenyl]-17β-hydroxy-17α-propinyl-4,9(10)-estradien-3-on (RU 38486) und

11β-(4-Dimethylaminophenyl)-17α-hydroxy-17β-(3-hydroxypropyl)-13α-methyl-4,9-gonadien-3-on

als Modellsubstanzen ausgewählt. Die geprüften Dosierungen sind der Tabelle 1 und den Abbildungen 1 bis 4 zu entnehmen.

1. Untersuchungen an graviden Meerschweinchen

1.1 Prüfung der Kombination
Versuchsbeschreibung:

Gravide Meerschweinchen mit einem Körpergewicht von ca. 800 g wurden am 42. Tag der Schwangerschaft in den Versuch genommen (der zweite Tag der Vaginalöffnung in der Anpaarungsphase wurde als erster Tag der Gravidität gerechnet). Vor Versuchsbeginn wurde die Gravidität durch Palpation kontrolliert. Die Behandlung mit den ausgewählten Prüfsubstanzen bzw. der Kombination erfolgte durch tägliche Injektion am 43. und 44. Tag der Gravidität. Die Testsubstanzen wurden dazu in Benzylbenzoat + Rizinusöl (Mischungsverhältnis bei Suproston: 1 + 2; RU 38486: 2 + 4,5) gelöst und die tägliche Dosis in einem Volumen von 0,4 ml (Sulproston) bzw. 1,0 ml (RU 38486) s.c. injiziert. Das eventuelle Ausstossen von Früchten wurde unter und nach der Behandlung mehrfach täglich kontrolliert. Am 50. Tag der Gravidität wurden die Tiere getötet. Die Uteri wurden inspiziert und die Foeten befundet.

Ergebnisse:

Die Ergebnisse der Versuche zur Abortinduktion beim graviden Meerschweinchen bei kombinierter Verabreichung von Antigestagen und Prostaglandin sind in der Tabelle 1 zusammengefasst.

Sulproston:

Die abortauslösende Wirkung von Sulproston war dosisabhängig. Bei einer Dosis von 0,03 mg/d s.c. wurde eine Abortrate von 30% (= Abort bei 3 von 10 behandelten Tieren) ermittelt. Die Ausstossung von Embryonen aus dem Uterus erfolgte bei dieser Dosis mit einer Latenz von ca. 1–2 Tagen (s. Abbildung 1).

Antigestagene:

Mit dem Antigestagen RU 38486 war eine Unterbrechung einer bestehenden Schwangerschaft mit 30 mg/d s.c. bei 4 von 9 behandelten Tieren zu erzielen. Bei einer Dosis von 10,0 mg/d s.c. lag die Abortrate bei 3/9 behandelten Tieren. Nach 3,0 mg/d s.c. abortierte nur noch 1 von 8 behandelten Tieren. Die Aborte erfolgten mit einer 4- bis 7tägigen Latenz vom Behandlungsbeginn (siehe Abbildung 1).

AG/PG-Kombination:

Die Kombination von subabortiven Antigestagendosen (3,0 mg bzw. 10,0 mg RU 38486/d s.c.) mit einer marginal wirksamen Sulprostondosis von 0,03 mg/d s.c. führte im Vergleich zur alleinigen Antigestagen-Behandlung jeweils zu einer deutlich höheren Abortrate und zu einem weit schnelleren Auftreten der Aborte. Das Induktions-Abortintervall war auch kürzer als bei alleiniger PG-Behandlung, sofern diese überhaupt die Ausstossung der Fruchtanlagen bewirkte (siehe Tabelle 1 und Abbildung 1).

1.2 Prüfung bei sequentieller Behandlung
Versuchsbeschreibung:

Gravide Meerschweinchen mit einem Körpergewicht von ca. 800 g wurden am 42. Tag der Schwangerschaft in den Versuch genommen (der zweite Tag der Vaginalöffnung in der Anpaarungsphase wurde als erster Tag der Gravidität gerechnet). Vor Versuchsbeginn wurde die Gravidität durch Palpation kontrolliert. Die Behandlung mit den ausgewählten Antigestagenen erfolgte durch tägliche Injektion am 43. und 44. Tag der Gravidität. Das Prostaglandin wurde am 45. Tag appliziert. Das Antigestagen wurde dazu in Benzylbenzoat + Rizinusöl (Mischungsverhältnis 2 + 4,5) gelöst und die tägliche Dosis in einem Volumen von 1,0 ml subcutan injiziert. Das Sulproston wurde in der galenischen Zubereitung der Nalador[R]-Ampulle verwendet und subcutan injiziert. Das eventuelle Ausstossen von Früchten wurde unter und nach der Behandlung mehrfach täglich kontrolliert. Am 50. Tag der Gravidität wurden die Tiere getötet. Die Uteri wurden inspiziert und die Foeten befundet.

Ergebnisse:

Die Ergebnisse der Versuche zur Abortinduktion beim graviden Meerschweinchen bei sequentieller Anwendung von Antigestagen und Prostaglandin sind in der Abbildung 2 zusammengefasst.

Sulproston:

Bei einer Dosis von 0,1 mg Sulproston/d s.c. wurde eine Abortrate von 50% (= Abort bei 3 von 6 behandelten Tieren) ermittelt. Die Ausstossung von Embryonen aus dem Uterus erfolgte bei dieser Dosis mit einer Latenz von ca. 6–24 Stunden (s. Abbildung 2).

Antigestagene:

Mit dem Antigestagen RU 38486 war eine Unterbrechung einer bestehenden Schwangerschaft mit 10 mg/d s.c. bei 3 von 9 behandelten Tieren zu erzielen. Allerdings erfolgte der Abort erst am Tag 48 bzw. 49, d.h. mit einer 5- bis 6tägigen Latenz vom Behandlungsbeginn (siehe Abbildung 2).

AG/PG-Sequenzbehandlung:

Bei sequentieller Verabreichung der obengenannten marginal wirksamen AG- und PG-Dosen kam es bei allen behandelten Meerschweinchen (6/6 Tieren) zum Abbruch der Gravidität (siehe Abbildung 2), wobei die zeitliche Latenz viel geringer als bei der alleinigen PG-Behandlung war, sofern diese überhaupt erfolgreich war (Medienwerte: 4 Stunden versus 12 bis 24 Stunden).

2. Untersuchungen an graviden Ratten

Versuchsbeschreibung:

Die Versuche wurden an weiblichen Wistar-Ratten hauseigener Zucht im Gewicht von ca. 200 g durchgeführt. Nach erfolgter Anpaarung wurde der Schwangerschaftsbeginn durch Nachweis von Spermien in Vaginalabstrichen gesichert.

Der Tag des Spermiennachweises gilt als Tag 1 der Gravidität (= d1 p.c.).

Die Antigestagene wurden in einem Benzylbenzoat-Rizinusöl-Gemisch (Verhältnis 1+4) gelöst. Das Vehikelvolumen pro Einzeldosis betrug 0,2 ml. Die Behandlung erfolgte subcutan.

Die gewählten Dosierungen sind der Abbildung 3 zu entnehmen.

Das Prostaglandin Sulproston wurde in einem Gemisch aus Äthanol + Benzylbenzoat + Rizinusöl (Verhältnis 1+5+12) gelöst. Das Vehikelvolumen der gewählten Einzeldosis von 0,1 mg betrug 0,2 ml. Sulproston wurde subcutan appliziert.

Vor Versuchsbeginn wurde die Gravidität durch Palpation kontrolliert. Die Zuordnung der graviden Tiere zu den verschiedenen Versuchsgruppen erfolgte zufällig (n = 6/Gruppe). Bei alleiniger Gabe der ausgewählten Antigestagene erfolgte die Behandlung durch tägliche Injektion von d13–15 der Schwangerschaft. Gruppen, die einer kombinierten Antigestagen-/Prostaglandin-Behandlung zugeführt wurden, erhielten zusätzlich 2 × 0,1 mg Sulproston/Tier s.c. an d15 p.c.

Einer weiteren Gruppe wurde allein Sulproston verabreicht (2 × 0,1 mg/Tier s.c. an d15 p.c.). Der Kontrollgruppe wurde von d13–15 p.c. täglich 0,2 ml des Benzylbenzoat-Rizinusöl-Vehikels appliziert. An d17 p.c. wurden die Ratten getötet und die Uteri auf lebende und tote Foeten, retinierte Placenten sowie leere Nidationsstellen hin untersucht. Von allen Uteri wurden Fotos angefertigt. Pro Gruppe wurde der %-Satz kompletter Aborte (per Definition: leere Nidationsstelle) berechnet.

Ergebnisse:

Die Ergebnisse der Versuche zur Abortinduktion bei graviden Ratten sind in den Abbildungen 3 und 4 dokumentiert.

Die Behandlung mit wirksamen Antigestagenen führte bei Ratten zur Auslösung von Aborten. Es besteht allerdings eine Tendenz zu inkompletten Aborten, z.T. gehen die Aborte mit länger anhaltenden vaginalen Blutungen einher. (Ein entsprechendes Verhalten wurde in ersten klinischen Untersuchungen mit RU-486 zum Zeitpunkt der ausgebliebenen Menstruation beobachtet).

Für die Testsubstanz

11β-[(4-N,N-Dimethylamino)-phenyl]-17α-hydroxy-17β-(3-hydroxypropyl)-13α-methyl-4,9-gonadien-3-on

(A) betrug der %-Satz kompletter Aborte bei 3tägiger s.c.-Gabe von 3,0 mg/d s.c. 49,5%. Nach Verabreichung von 3,0 mg RU-486/d s.c. lag die Rate kompletter Aborte bei 8,3%. Die zusätzliche Sulprostongabe von 2 × 0,1 mg/d s.c. an Tag 15 konnte die abortive Wirkung von 11β-[(4-N,N-Dimethylamino)-phenyl]-17α-hydroxy-17β-(3-hydroxypropyl)-13α-methyl-4,9-gonadien-3-on

und RU-486 deutlich steigern (siehe Abb. 3 und 4).

Tabelle 1

Vergleichende Betrachtung der abortiven Wirkung von Sulproston (PG), RU-38.486 (kompetitiver Progesteronantagonist) und der Kombination beider Substanzen beim graviden Meerschweinchen.[1] – Behandlung d43 und d44, Autopsie an d50.

| Dosis mg/d s.c. | n Tiere mit Abort / n Tiere gesamt, ( )* = 'missed abortions' | | |
| | Sulproston | RU-38.486 | S-/RU-Kombination |
| --- | --- | --- | --- |
| 30,0 | – | 4/9 | 0,03 mg Sulproston + 10,0 mg RU-38.486 } 7/9 |
| 10,0 | – | 3/9 | |
| 3,0 | – | 1/8 | |
| 1,0 | – | | 0,03 mg Sulproston + 3,0 mg RU-38.486 } 4/8 |
| 0,3 | 10/10 | | |
| 0,1 | 8/10 (2/10)* | | |
| 0,03 | 3/10 | | |
| 0,01 | 0/9 | | |

[1] Screenin-Daten des Dept. Endokrinpharmakologie I, Schering AG

Behandlung: d 43 + d 44
Autopsie:    d 50

◯   RU-486     30,0   mg/d s.c. (4/9)

⊘   RU-486     10,0   mg/d s.c. (3/9)

✜   RU-486      3,0   mg/d s.c. (1/8)

◼   Sulproston     0,03 mg/d s.c. (3/10)

✳   RU-486 + Sulproston     10,0 mg/d s.c. 0,03 mg/d s.c. (7/9)

△   RU-486 + Sulproston     3,0 mg/d s.c. 0,03 mg/d s.c. (4/8)

( )   = Abortrate

Abb. 1: Schwangerschaftsunterbrechung beim Meerschweinchen. – Tag des Abortes unter verschiedenen Behandlungen.

Abb. 2: Vergleichende Betrachtung der abortiven Wirkung von Sulproston (PG) und RU-38.486 (AG) und der sequentiellen Anwendung beider Substanzen beim graviden Meerschweinchen.
Behandlung AG: d43 und d44; PG: d45.
Applikation: s.c.

Abb. 3: Auswertung der synergistischen Antigestagen (AG)/Suprostone (S) – Wirkung bei fortgeschrittener Gravidität der Ratte. – Antigestagen: 3,0 mg/d s.c. Tage 13–15 p.c., Sulproston: 0,1 mg 2 × Tag 15 p.c., Autopsie Tag 17 p.c.

RU-486 = 11β-(4-N,N-Dimethylaminophenyl)-17β-hydroxy-17α-propinyl-4,9(10)-estradien-3-on
A      = 11β-(4-Dimethylaminophenyl)-17α-hydroxy-17β-(3-hydroxypropyl)-13α-methyl-4,9-gonadien-3-on

Abb. 4: Auswertung der synergistischen Antigestagen (AG)/Sulprostone(S) – Wirkung bei fortgeschrittener Gravidität der Ratte. – Antigestagen: 3.0 mg/d s.c. Tage 13–15 p.c., Sulprostone: 0.1 mg 2 × Tag 15 p.c., Autopsie Tag 17 p.c.

11β-(4-N,N-Dimethylaminophenyl)-17β-hydroxy-
17α-propinyl-4,9(10)-estradien-3-on            RU-486

RU-486 + S

11β-(4-Dimethylaminophenyl)-17α-hydroxy-17β-
(3-hydroxypropyl)-13α-methyl-4,9-gonadien-3-on

11β-(4-Dimethylaminophenyl)-17α-hydroxy-17β-
(3-hydroxypropyl)-13α-methyl-4,9-gonadien-3-on
            + S

Kontrolle

Sulproston

Abb. 4: Auswertung der synergistischen Antigestagen (AG)/Sulprostone(S) – Wirkung bei fortgeschrittener Gravidität der Ratte. – Antigestagen: 3.0 mg/d s.c. Tage 13–15 p.c., Sulprostone: 0.1 mg 2 × Tag 15 p.c., Autopsie Tag 17 p.c.

11β-(4-N,N-Dimethylaminophenyl)-17β-hydroxy-
17α-propinyl-4,9(10)-estradien-3-on     RU-486

RU-486 + S

11β-(4-Dimethylaminophenyl)-17α-hydroxy-17β-
(3-hydroxypropyl)-13α-methyl-4,9-gonadien-3-on

11β-(4-Dimethylaminophenyl)-17α-hydroxy-17β-
(3-hydroxypropyl)-13α-methyl-4,9-gonadien-3-on + S

Kontrolle

Sulproston

0 139 608

## Patentansprüche für die Vertragsstaaten: BE, CH, DE, FR, GB, IT, LI, LU, NL, SE

1. Kombinationserzeugnis zur gemeinsamen Verwendung zur Geburtseinleitung oder für den Schwangerschaftsabbruch, enthaltend ein Prostaglandin (PG) und ein Antigestagen (AG).

2. Erzeugnis gemäss Anspruch 1, dadurch gekennzeichnet, dass Prostaglandin und Antigestagen in einem Gewichtsverhältnis 1:20 bis 1:6000 stehen.

3. Erzeugnis gemäss Anspruch 1, dadurch gekennzeichnet, dass Prostaglandin und Antigestagen in getrennten Dosiseinheiten vorliegen.

4. Erzeugnis gemäss Anspruch 1, dadurch gekennzeichnet, dass eine PG-Dosiseinheit 0,03–0,5 mg Sulproston (16-Phenoxy-ω-17,18-19,20-tetranor-PGE$_2$-methylsulfonylamid) oder eine biologisch äquivalente Menge eines anderen Prostaglandins enthält.

5. Erzeugnis gemäss Anspruch 1, dadurch gekennzeichnet, dass eine AG-Dosiseinheit 10–200 mg 11β-[(4-N,N-Dimethylamino)-phenyl]-17β-hydroxy-17α-propinyl-4,9(10)-estradien-3-on oder eine biologisch äquivalente Menge eines anderen Antigestagens enthält.

6. Verwendung von Prostaglandinen und Antigestagenen für die Herstellung von Arzneimitteln zur gleichzeitigen, getrennten oder zeitlich abgestuften Anwendung zur Geburtseinleitung oder für den Schwangerschaftsabbruch.

7. Verwendung für die Herstellung von Arzneimitteln nach Anspruch 6, dadurch gekennzeichnet, dass Prostaglandin und Antigestagen in einem Gewichtsverhältnis von 1:20 bis 1:6000 stehen.

8. Verwendung von 0,03–0,5 mg Sulproston als Prostaglandin oder einer biologisch äquivalenten Menge eines anderen Prostaglandins für die Herstellung von Arzneimitteln gemäss Anspruch 6.

9. Verwendung von 10–200 mg 11β-[(4-N,N-Dimethylamino)-phenyl]-17β-hydroxy-17α-propinyl-4,9(10)-estradien-3-on als Antigestagen oder einer biologisch äquivalenten Menge eines anderen Antigestagens für die Herstellung von Arzneimitteln gemäss Anspruch 6.

10. Verwendung von Prostaglandinen für die Herstellung von Arzneimitteln zur Geburtseinleitung in Kombination mit Antigestagenen.

11. Verwendung von Prostaglandinen für die Herstellung von Arzneimitteln für den Schwangerschaftsabbruch in Kombination mit Antigestagenen.

## Patentansprüche für den Vertragsstaat: AT

1. Verfahren zur Herstellung eines Mittels zur Verwendung für die Geburtseinleitung oder für den Schwangerschaftsabbruch, dadurch gekennzeichnet, dass man ein Prostaglandin (PG) und ein Antigestagen (AG) jeweils für sich oder gemeinsam mit den in der galenischen Pharmazie üblichen Zusätzen, Trägersubstanzen und/oder Geschmackskorrigentien vermischt und in eine geeignete Applikationsform überführt, wobei PG und AG in einem Gewichtsverhältnis von 1:20 bis 1:6000 stehen.

2. Verfahren gemäss Anspruch 1, dadurch gekennzeichnet, dass als PG-Dosiseinheit 0,03–0,5 mg Sulproston (16-Phenoxy-ω-17,18,19,20-tetranor-PGE$_2$-methylsulfonylamid) oder eine biologisch äquivalente Menge eines anderen Prostaglandins eingesetzt werden.

3. Verfahren gemäss Anspruch 1, dadurch gekennzeichnet, dass als AG-Dosiseinheit 10–200 mg 11β-[(4-N,N-Dimethylamino)-phenyl]-17β-hydroxy-17α-propinyl-4,9(10)-estradien-3-on oder eine biologisch äquivalente Menge eines anderen Antigestagens eingesetzt werden.

4. Verwendung von Prostaglandinen und Antigestagenen für die Herstellung von Arzneimitteln zur gleichzeitigen, getrennten oder zeitlich abgestuften Anwendung zur Geburtseinleitung oder für den Schwangerschaftsabbruch.

5. Verwendung für die Herstellung von Arzneimitteln nach Anspruch 6, dadurch gekennzeichnet, dass Prostaglandin und Antigestagen in einem Gewichtsverhältnis von 1:20 bis 1:6000 stehen.

6. Verwendung von 0,03–0,5 mg Sulproston als Prostaglandin oder einer biologisch äquivalenten Menge eines anderen Prostaglandins für die Herstellung von Arzneimitteln gemäss Anspruch 6.

7. Verwendung von 10–200 mg 11β-[(4-N,N-Dimethylamino)-phenyl]-17β-hydroxy-17α-propinyl-4,9(10)-estradien-3-on als Antigestagen oder einer biologisch äquivalenten Menge eines anderen Antigestagens für die Herstellung von Arzneimitteln gemäss Anspruch 6.

8. Verwendung von Prostaglandinen für die Herstellung von Arzneimitteln zur Geburtseinleitung in Kombination mit Antigestagenen.

9. Verwendung von Prostaglandinen für die Herstellung von Arzneimitteln für den Schwangerschaftsabbruch in Kombination mit Antigestagenen.

## Claims for the contracting states: BE, CH-LI, DE, FR, GB, IT, LU, NL, SE

1. Combination product for joint use for inducing labour or terminating pregnancy, containing a prostaglandin (PG) and an antigestagen (AG).

2. Product according to claim 1, characterised in that the ratio by weight of the prostaglandin to the antigestagen is from 1:20 to 1:6000.

3. Product according to claim 1, characterised in that the prostaglandin and antigestagen are in separate dosage units.

4. Product according to claim 1, characterised in that a PG dosage unit contains from 0.03 to 0.5 mg of Sulprostone (16-phenoxy-ω-17,18,19,20-tetranor-PGE$_2$-methyl-sulphonylamide) or a biologically equivalent amount of another prostaglandin.

5. Product according to claim 1, characterised in that an AG dosage unit contains from 10 to 200 mg of

11β-[(14-N,N-dimethylamino)-phenyl]-17β-hydroxy-17α-propynyl-4,9,(10)-oestradien-3-one or a biologically equivalent amount of another antigestagen.

6. Use of prostaglandins and antigestagens for the manufacture of medicaments for simultaneous, separate or time-staggered administration for inducing labour or terminating pregnancy.

7. Use for the manufacture of medicaments according to claim 6, characterised in that the ratio by weight of the prostaglandin to the antigestagen is from 1:20 to 1:6000.

8. Use of from 0.03 to 0.5 mg of Sulprostone as the prostaglandin, or of a biologically equivalent amount of another prostaglandin, for the manufacture of medicaments according to claim 6.

9. Use of from 10 to 200 mg of

11β-[4-N,N-dimethylamino)-phenyl]-17β-hydroxy-17α-propynyl-4,9(10)-oestradien-3-one as the antigestagen, or of a biologically equivalent amount of another antigestagen, for the manufacture of medicaments according to claim 6.

10. Use of prostaglandins for the manufacture of medicaments for inducing labour in combination with antigestagens.

11. Use of prostaglandins for the manufacture of medicaments for terminating pregnancy in combination with antigestagens.

### Claims for the contracting state: AT

1. Process for the manufacture of an agent for use in inducing labour or terminating pregnancy, characterised in that a prostaglandin (PG) and an antigestagen (AG) are each individually, or together, mixed with additives, carriers and/or taste correctives customary in galenical pharmacy, and converted into a suitable form of administration, the ratio by weight of PG to AG being from 1:20 to 1:6000.

2. Process according to claim 1, characterised in that the PG dosage unit used is from 0.03 to 0.5 mg of Sulprostone

(16-phenoxy-ω-17,18,19,20-tetranor-PGE₂-methylsulphonylamide) or a biologically equivalent amount of another prostaglandin.

3. Process according to claim 1, characterised in that the AG dosage unit used is from 10 to 200 mg of

11β-[(4-N,N-dimethylamino)-phenyl]-17β-hydroxy-17α-propynyl-4,9,(10)-oestradien-3-one or a biologically equivalent amount of another antigestagen.

4. Use of prostaglandins and antigestagens for the manufacture of medicaments for simultaneous, separate or time-staggered administration for inducing labour or terminating pregnancy.

5. Use for the manufacture of medicaments according to claim 4, characterised in that the ratio by weight of the prostaglandin to the antigestagen is from 1:20 to 1:6000.

6. Use of from 0.03 to 0.5 mg of Sulprostone as the prostaglandin, or of a biologically equivalent amount of another prostaglandin, for the manufacture of medicaments according to claim 4.

7. Use of from 10 to 200 mg of

11β-[(4-N,N-dimethylamino)-phenyl]-17β-hydroxy-17α-propynyl-4,9(10)-oestradien-3-one as the antigestagen, or of a biologically equivalent amount of another antigestagen, for the manufacture of medicaments according to claim 4.

8. Use of prostaglandins for the manufacture of medicaments for inducing labour in combination with antigestagens.

9. Use of prostaglandins for the manufacture of medicaments for terminating pregnancy in combination with antigestagens.

### Revendications pour les Etats contractants: BE, CH-LI, DE, FR, GB, IT, LU, NL, SE

1. Produit comportant une association de substances à utiliser en commun pour déclencher un accouchement ou pour interrompre une grossesse, produit qui contient une prostaglandine (PG) et un anti-progestatif (AP).

2. Produit selon la revendication 1 caractérisé en ce que la prostaglandine et l'anti-progestatif sont dans un rapport pondéral compris entre 1:20 et 1:6000.

3. Produit selon la revendication 1 caractérisé en ce que la prostaglandine et l'anti-progestatif se trouvent dans des unités de prise séparées.

4. Produit selon la revendication 1 caractérisé en ce qu'une unité de prise de PG contient de 0,03 à 0,5 mg de sulproston (c'est-à-dire de méthylsulfonylamide de la phénoxy-16ω-tétranor-17,18,19,20 PGE₂ ou une quantité biologiquement équivalente d'une autre prostaglandine.

5. Produit selon la revendication 1 caractérisé en ce qu'une unité de prise d'AP contient de 10 à 200 mg de (diméthylamino-4 phényl)-11β hydroxy-17β propynyl-17α oestradiène-4,9(10) one-3 ou une quantité équivalente d'un autre anti-progestatif.

6. Application de prostaglandines et d'anti-progestatifs pour la préparation de médicaments destinés à l'application simultanée, séparée ou graduelle dans le temps, pour le déclenchement d'un accouchement ou pour l'interruption d'une grossesse.

7. Application pour la préparation de médicaments selon la revendication 6, caractérisé en ce que la prostaglandine et l'anti-progestatif sont dans un rapport pondéral compris entre 1:20 et 1:6000.

8. Application de 0,03 à 0,5 mg de sulproston comme prostaglandine ou d'une quantité biologiquement équivalente d'une autre prostaglandine pour la préparation de médicaments selon la revendication 6.

9. Application de 10 à 200 mg de (diméthylamino-4-phényl)-11β hydroxy-17β propynyl-17α oestradiène-4,9(10) one-3 comme anti-progestatif ou d'une quantité biologiquement équivalente d'un autre anti-progestatif pour la préparation de médicaments selon la revendication 6.

10. Application de prostaglandines pour la préparation de médicaments destinés à être utilisés, en association avec des anti-progestatifs, pour le déclenchement d'un accouchement.

11. Application de prostaglandines pour la préparation de médicaments destinés à être utilisés, en associations aves des anti-progestatifs, pour l'interruption d'une grossesse.

**Revendications pour l'Etat contractant: AT**

1. Procédé de préparation d'un produit destiné à être utilisé pour déclencher un accouchement ou pour interrompre une grossesse, procédé caractérisé en ce qu'on mélange une prostaglandine (PG) et un anti-progestatif (AP), chacun isolément ou en commun, avec les additifs, excipients et/ou correcteurs de goût couramment utilisés en pharmacie et on met les mélanges sous une forme convenant pour l'administration, la prostaglandine et l'anti-progestatif étant présents dans un rapport pondéral compris entre 1:20 et 1:6000.

2. Procédé selon la revendication 1 caractérisé en ce qu'une unité de prise de PG contient de 0,03 à 0,5 mg de sulproston (c'est-à-dire de méthyl-sulfonylamide de la phénoxy-16ω-tétranor-17,18,19,20 PGE$_2$) ou une quantité biologiquement équivalente d'une autre prostaglandine.

3. Procédé selon la revendication 1 caractérisé en ce qu'une unité de prise d'AP contient de 10 à 200 mg de (diméthylamino-4 phényl)-11-β-hydroxy-17β-propynyl-17α-oestradiène-4,9(10) one-3

ou une quantité équivalente d'un autre anti-progestatif.

4. Application de prostaglandines et d'antiprogestatifs pour la préparation de médicaments destinés à l'application simultanée, séparée ou graduelle dans le temps, pour le déclenchement d'un accouchement ou pour l'interruption d'une grossesse.

5. Application pour la préparation de médicaments selon la revendication 4, caractérisée en ce que la prostaglandine et l'anti-progestatif sont dans un rapport pondéral compris entre 1:20 et 1:6000.

6. Application de 0,03 à 0,5 mg de sulproston comme prostaglandine ou d'une quantité biologiquement équivalente d'une autre prostaglandine pour la préparation de médicaments selon la revendication 4.

7. Application de 10 à 200 mg de (diméthylamino-4-phényl)-11β hydroxy-17β propynyl-17α oestradiène-4,9(10) one-3 comme anti-progestatif ou d'une quantité biologiquement équivalente d'un autre anti-progestatif pour la préparation de médicaments selon la revendication 4.

8. Application de prostaglandines pour la préparation de médicaments destinés à être utilisés, en association avec des anti-progestatifs, pour le déclenchement d'un accouchement.

9. Application de prostaglandines pour la préparation de médicaments destinés à être utilisés, en association avec des anti-progestatifs, pour l'interruption d'une grossesse.